# EUROPEAN PATENT APPLICATION

(11) **EP 1 348 462 A1**
(43) Date of publication of application: **01.10.2003**
(21) Application number: 03075803.1
(22) Date of filing: 20.03.2003
(51) Int. Cl.: A61M 25/06, A61M 5/50

(54) **Needle assembly**

(30) Priority: 20.03.2002 US 365922 P
(71) Applicant: Becton, Dickinson and Company, Franklin Lakes, New Jersey 07417 (US)
(72) Inventor: Swenson, Kirk D., North Caldwell, NJ 07006 (US); Niermann, Volker, Bound Brook, NJ 08805 (US)
(74) Representative: 't Jong, Bastiaan Jacobus

(57) **Abstract**

The shieldable needle assembly includes a needle cannula and a hub member supporting a proximal end of the needle cannula. A shield member is aligned with the hub member and extends co-axially about the needle cannula. The shield member is movable between a retracted position in which a puncture tip of the needle cannula is exposed, and an extended position covering the puncture tip. A pair of extendable members is connected between the hub and the shield members. The extendable members each include a pair of folding legs. A tension spring is connected between the extendable members. The hub member is movable in an axial direction away from the shield member.

## Description

### CROSS REFERENCE TO RELATED APPLICATIONS

This application claims the benefit of U.S. Provisional Application No. 60/365,922 filed on March 20, 2002.

### BACKGROUND OF THE INVENTION

### 1. Field of the Invention

The present invention relates to blood collection sets for safe and convenient handling of needles. More particularly, the present invention relates to a blood collection set including a needle assembly having a low profile, forward moving safety shield for protection from a used needle tip.

### 2. Description of Related Art

Disposable medical devices having medical needles are used for administering medication or withdrawing fluid from the body of a patient. Such disposable medical devices typically include blood collecting needles, fluid handling needles, and assemblies thereof. Current medical practice requires that fluid containers and needle assemblies used in such devices be inexpensive and readily disposable. Consequently, existing blood collection devices typically employ some form of durable, reusable holder on which detachable and disposable medical needles and fluid collection tubes may be mounted. A blood collection device of this nature may be assembled prior to use and then disassembled after use. Thus, these blood collection devices allow repeated use of a relatively expensive holder upon replacement of relatively inexpensive medical needles and/or fluid collection tubes. In addition to reducing the cost of collecting blood specimens, these blood collection devices help minimize the production of hazardous waste material.

A blood collection device or intravenous (IV) infusion device typically includes a needle cannula having a proximal end, a pointed distal end, and a lumen extending therebetween. The proximal end of the needle cannula is securely mounted in a plastic hub defining a central passage that communicates with the lumen extending through the needle cannula. A thin, flexible thermoplastic tube is connected to the hub and communicates with the lumen of the needle cannula. The end of the plastic tube remote from the needle cannula may include a fixture for connecting the needle cannula to a blood collection tube or other receptacle. The specific construction of the fixture will depend upon the characteristics of the receptacle to which the fixture is to be connected.

In order to reduce the risk of incurring an accidental needle-stick wound, protection of used needle cannulas becomes important. With concern about infection and transmission of diseases, methods and devices to enclose or cover the used needle cannula have become very important and in great demand in the medical field. For example, needle assemblies commonly employ a safety shield that can be moved into shielding engagement with a used needle cannula to minimize risk of an accidental needle-stick.

Some needle safety shields are referred to as "tip guards" and include a small rigid guard that may be telescoped along the length of the needle cannula and extended over the pointed distal end of the needle cannula for protection. Such conventional tip guards may include some form of tether for limiting the travel of the tip guard to the length of the needle cannula. An example of the foregoing is disclosed by U.S. Patent No. 5,176,655 to McCormick et al. The McCormick et al. patent discloses the use of flexible loop-like straps for limiting the distal movement of a tip guard.

Needle shields that incorporate movable tip guards are typically manually actuated. For example, U.S. Patent Nos. Re 36,447 and Re 36,398, both to Byrne et al., disclose a safety device for a hypodermic needle that includes a plastic sheath, which is used to cover the puncture tip of the needle. The plastic sheath incorporates a thumb guard, which the user of the safety device may grasp to move the plastic sheath to a position covering the puncture tip of the needle. U.S. Patent No. 5,951,525 to Thorne et al. discloses a manually operated safety needle apparatus that includes two pairs of opposed legs adapted to move the tip guard of the apparatus to a position covering the used needle cannula. U.S. Patent Nos. 5,562,637 and 5,562,636, both to Utterburg, disclose a rectangular needle protector sheath for use with a needle cannula that may be extended over the needle cannula after its use. Other prior art devices, such as those disclosed by U.S. Patent Nos. 5,290,264 to Utterberg and 5,192,275 to Burns, provide "grippable" members attached to the tip guards to facilitate moving the tip guards to a position covering the puncture tip of a needle cannula. In addition to providing gripping members for moving the tip guards, prior art devices in this area often include flexible wings, which are used as means for securing the needle assemblies to the body of a patient during a medical procedure. Examples of "winged" needle assemblies may be found in U.S. Patent Nos. 5,120,320 to Fayngold; and 5,154,699; 5,088,982; and 5,085,639 all to Ryan. Other prior art in this area includes U.S. Patent Nos. 5,266,072 and 5,112,311, both to Utterberg et al., which also disclose guarded winged needle assemblies.

Conventional tip guards, such as those discussed hereinabove, often include a structure that lockingly engages over the pointed distal end of the used needle cannula to prevent a re-exposure of the needle cannula. The structure for preventing the re-exposure of the needle cannula may include a metallic spring clip or a transverse wall formed integrally with one end of the tip guard. An example of a metallic spring clip is disclosed by the McCormick et al. patent discussed previously.

Conventional tip guards, such as those discussed hereinabove, often further require extensive mechanics for positioning the tip guard over the needle cannula. This results in complex arrangements that are costly to manufacture and assemble. Additionally, operation of the needle assemblies to move the tip guard into the proper position over the pointed distal end of the needle cannula requires substantial manual manipulation by the user of the device, exposing the user to potential needle-stick wounds.

In view of the foregoing, a need exists for a blood collection set including a shieldable needle assembly that achieves secure and effective shielding of a used needle cannula, which is simple and inexpensive to manufacture and easy to operate.

### SUMMARY OF THE INVENTION

The present invention is directed to a blood collection set incorporating a shield needle assembly. The blood collection set generally includes a fixture for connecting the blood collection set to a receptacle, a flexible tube, and the shieldable needle assembly. The flexible tube has opposed first and seconds ends, with the first end of the flexible tube connected to the fixture. The needle assembly used in the blood collection set includes a needle cannula having a proximal end and a distal end with a puncture tip. The proximal end of the needle cannula is connected to a hub member, which supports the proximal end of the needle cannula. The distal end of the needle cannula projects outward from the hub member. The needle cannula defines a lumen in fluid communication with the flexible tube and the fixture. The needle assembly includes a shield member having a housing defining a central bore. The shield member is in axial alignment with the hub member and extends co-axially about the needle cannula. The shield member and the hub member are adapted for relative axial movement with respect to each other in opposing axial directions between a first position in which the puncture tip of the needle cannula is exposed and a second position in which the shield member covers the puncture tip of the needle cannula. The needle assembly further includes a pair of extendable members connecting the hub member and the shield member. The extendable members each include a pair of folding legs connected by a hinged knee joint. One leg of the pair of legs for each extendable member is connected hingedly to the hub member at a hub hinge and the other leg of the pair of legs for each extendable member is connected hingedly to the shield member. A tension spring is operatively connected between the extendable members. The hub hinge is located in front of the tension spring when the shield member is in the retracted position. The hub member is movable in an axial direction away from a proximal end of the shield member. When the legs connected to the hub member for each extendable member are pivoted to a position forming an angle of greater than 90° with the axis of the needle cannula, the shield member and the hub member are biased in opposing axial directions to the second position by the tension spring.

The shield member may include a pair of butterfly wings extending laterally from opposing sides of the housing of the shield member. In addition, the housing of the shield member may further include a dorsal wing located between the butterfly wings.

The shield member may further include a tip guard for protectively surrounding the puncture tip of the needle cannula when the shield member is moved to the second position. The tip guard may comprise a tip guard housing and a spring clip connected to the tip guard housing. The spring clip may be biased against the needle cannula when the shield member is in the first position and resiliently extends over the puncture tip of the needle cannula when the shield member is in the second position.

Alternatively, the tip guard may be externally attached to the housing of the shield member. The externally secured tip guard may comprise a spring leg extending axially along the housing, a pair of clip legs securing the tip guard to the housing, and a locking plate resiliently biased against the needle cannula when the shield member is in the first position. The locking plate is configured to resiliently extend over a distal opening to the central bore of the housing when the shield member is moved to the second position.

The knee joint for each extendable member may further include a fingerplate to enable the user to manipulate the needle assembly. The needle assembly may further include a locking assembly extending from the fingerplate of at least one of the extendable members toward the needle cannula and configured to engage the needle cannula such that when the shield member is moved to the second position by the tension spring, the locking assembly automatically engages onto the needle cannula to secure the shield member in the second position. The locking assembly may be comprised of a pair of opposed locking members defining a recess therebetween for receiving the needle cannula. The locking members are preferably made of a resiliently flexible material such that the needle cannula is able to separate the locking members under force provided by the tension spring.

The locking assembly may be configured to connect the knee joints of the extendable members, and be comprised of a locking member having a plurality of ratchet teeth. The ratchet teeth of the locking member may be configured to permit unidirectional movement of the knee joints of the extendable members toward one another. The locking member may be connected between the fingerplates of the extendable members.

The hub member may be adapted for connection to the flexible tube of the blood collection set.

A removable protective sleeve may be positioned over the distal end of the needle cannula.

The present invention further includes a method of automatically placing a shieldable needle assembly into a safety state in which a puncture tip of a needle cannula is covered by the needle assembly. The method generally comprises the steps of providing the shieldable needle assembly as described hereinabove, using the shieldable needle assembly in a medical procedure, and moving the hub member in an axial direction away from a proximal end of the shield member until the legs connected to the hub member for each extendable member are pivoted to a position forming an angle of greater than 90° with the axis of the needle cannula such that the shield member and the hub member are biased in opposing axial directions to the second position by the tension spring. The method may further include the step of automatically engaging the locking members of the locking assembly onto the needle cannula when the shield member is biased to the second position by the tension spring.

Further details and advantages of the present invention will become apparent from the following detailed description when read in conjunction with the drawings.

### BRIEF DESCRIPTION OF THE DRAWINGS

FIG. 1 is a perspective view of a blood collection set including a shieldable needle assembly in accordance with the present invention and showing the needle assembly in a retracted position;

FIG. 2 is a cross-sectional view taken along line II-II in FIG. 1;

FIG. 3 is a cross-sectional view taken along line III-III in FIG. 1;

FIG. 4 is a perspective view of the shieldable needle assembly of FIG. 1 shown in a partially extended position;

FIG. 5 is a perspective view of the shieldable needle assembly of FIG. 1 shown in a further extended position;

FIG. 6 is a perspective view of the shieldable needle assembly of FIG. 1 shown in a fully extended position;

FIG. 7 is a side view of a tip guard used in the shieldable needle assembly of FIG. I shown prior to activation;

FIG. 8 is a side view of the tip guard of FIG. 7 shown in an activated, shielding position;

FIG. 9 is a perspective view of the shieldable needle assembly of FIG. 1 having a modified tip guard in accordance with the present invention;

FIG. 10 is a cross-sectional view taken along lines X-X in FIG. 9;

FIG. 11 is a cross-sectional view of the modified tip guard of FIG. 9 showing the tip guard covering a needle cannula of the shieldable needle assembly; and

FIG. 12 is a perspective view of the blood collection set and shieldable needle assembly is accordance with a second embodiment of the present invention.

### DETAILED DESCRIPTION

Referring to the drawings in which like reference characters refer to like parts throughout the several views thereof, the present invention is generally described in terms of a blood collection set and related features, and encompasses such a blood collection set as well as a shieldable needle assembly for use in such a blood collection set.

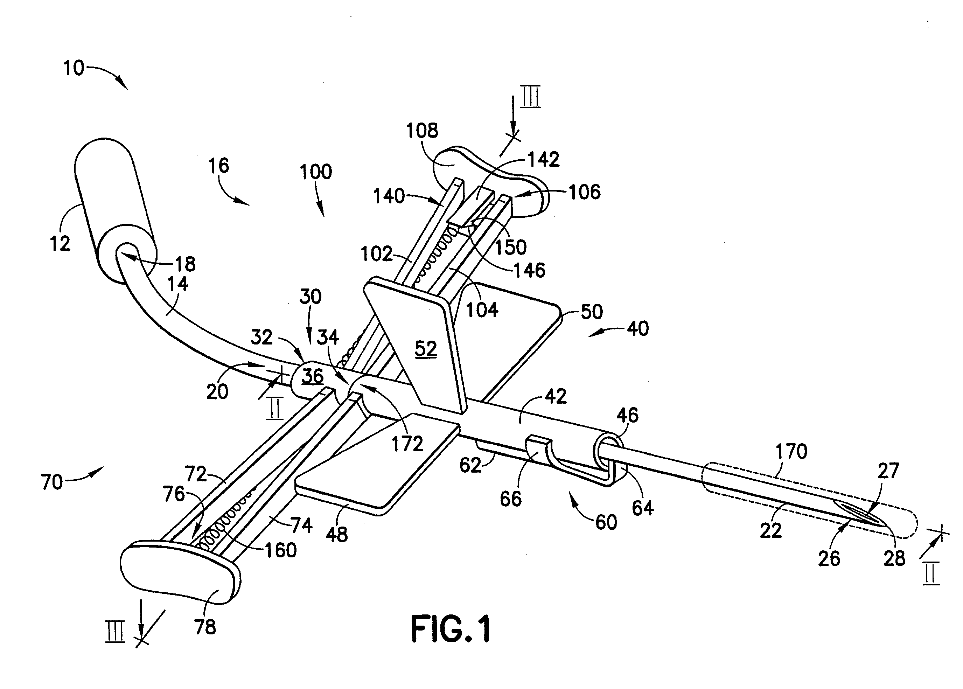

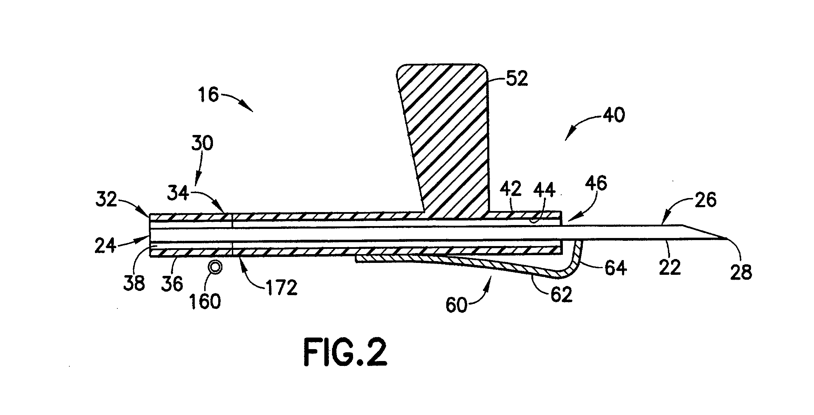

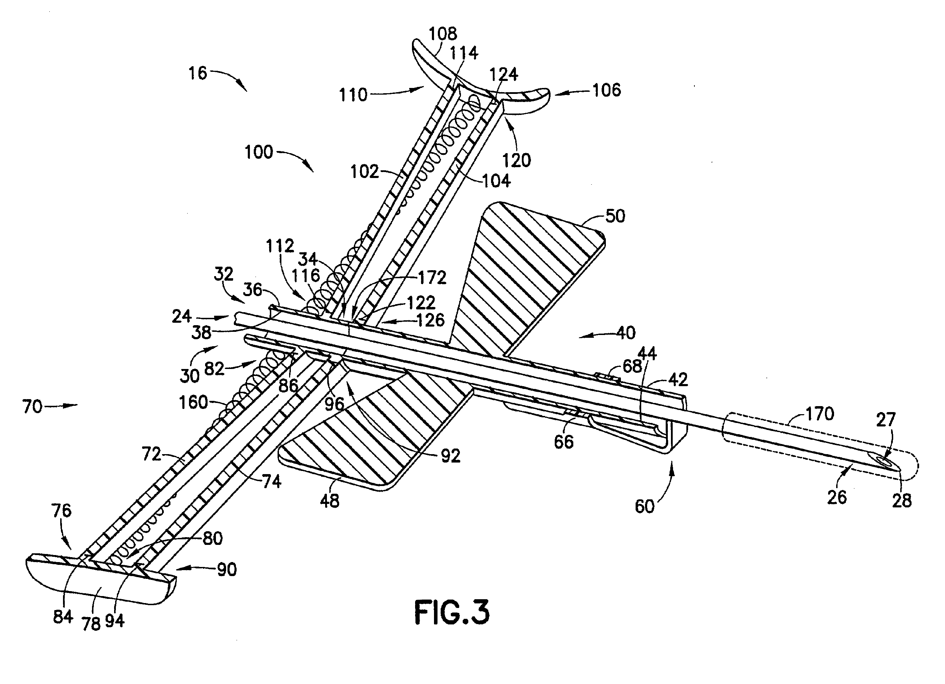

Referring to FIGS. 1-6, a blood collection set **10** in accordance with the present invention includes a fixture **12** for connecting the blood collection set **10** to a receptacle (not shown), a flexible tube **14**, and a shieldable needle assembly **16**. The flexible tube **14** has a first end **18** and a second end **20**. The first end **18** of the flexible tube **14** is connected to the fixture **12**. The first end **18** of the flexible tube **14** may be connected to the fixture **12** by means customary in the art.

The shieldable needle assembly **16** of the blood collection set **10** includes a needle cannula **22**. The needle cannula **22** has a proximal end **24** and an opposing distal end **26**. The needle cannula **22** defines a lumen **27** extending through the needle cannula **22** from the proximal end **24** to the distal end **26**. The distal end **26** of the needle cannula **22** is beveled to define a sharp puncture tip **28**, such as an intravenous (IV) puncture tip. The puncture tip **28** is provided for insertion into a patient's blood vessel, such as a vein, and is, therefore, designed to provide ease of insertion and minimal discomfort during venipuncture.

The shieldable needle assembly **16** of the blood collection set **10** includes a hub member **30**, a shield member **40**, and a pair of extendable members **70**, **100** connecting the hub member **30** and the shield member **40**. The hub member **30**, shield member **40**, and extendable members **70**, **100** may be integrally formed as a unitary body, which is desirably molded from a thermoplastic material. Alternatively, the hub member **30**, shield member **40**, and extendable members **70**, **100** may be separate parts, which are preferably molded from thermoplastic material.

The hub member **30** has a proximal end **32** and a distal end **34** and is generally defined as a rigid tubular wall **36** extending from the proximal end **32** to the distal end **34**. The tubular wall **36** defines an internal passage **38** extending from the proximal end **32** to the distal end **34**. The hub member **30** is adapted to support the proximal end **24** of the needle cannula **22**. In particular, the needle cannula **22** is positioned within the internal passage **38** defined by the hub member **30** and extends outward from the distal end **34** of the hub member **30**. Preferably, the needle cannula **22** and hub member **30** are formed as separate parts that are fixedly attached and secured through an appropriate medical grade adhesive, by direct mechanical attachment, or other similar means.

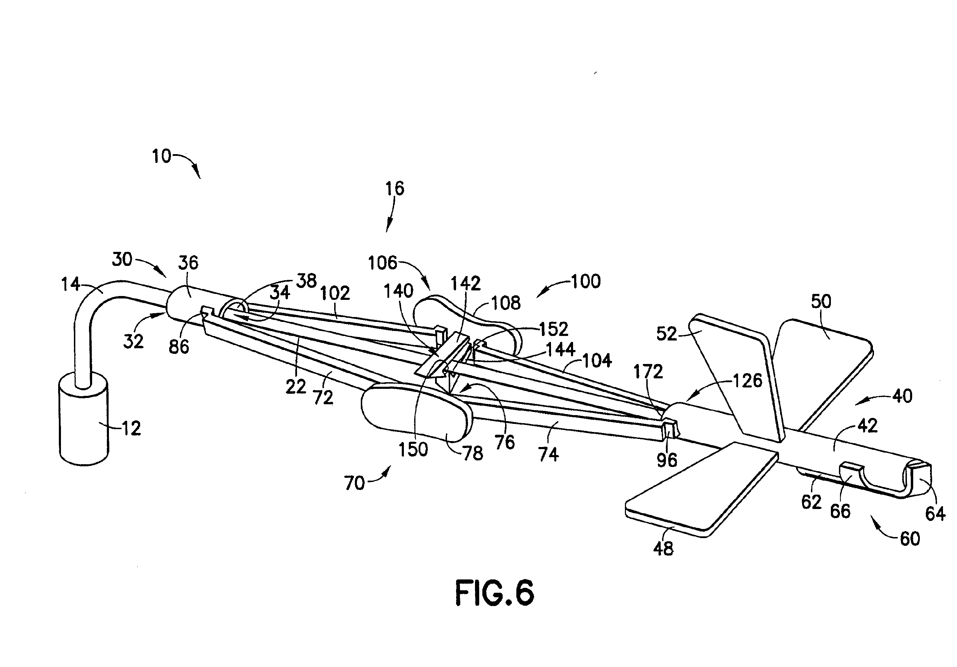

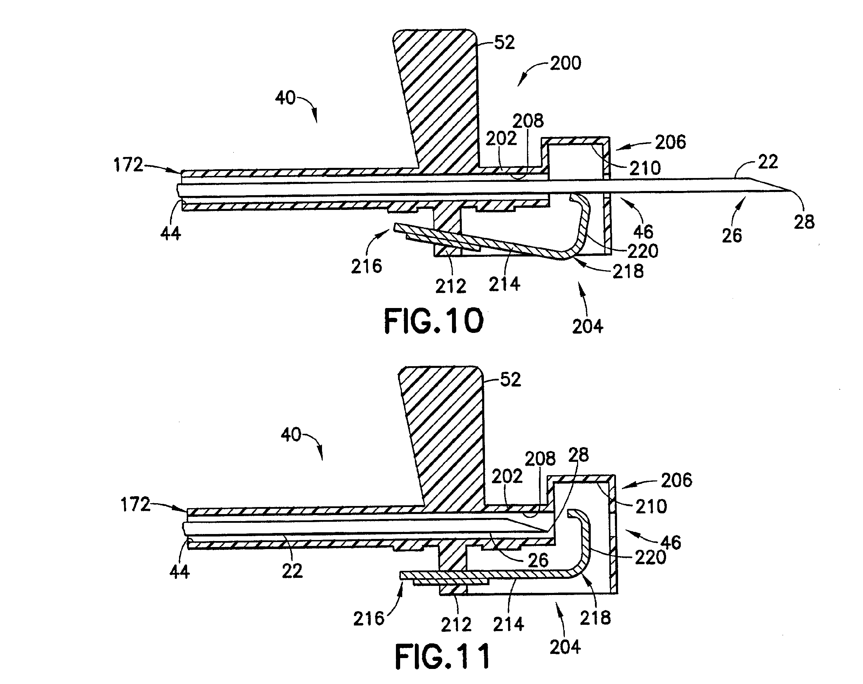

The shield member **40** and the hub member **30** are movable in opposing axial directions with respect to each other between a first position in which the shield member **40** is retracted and located adjacent and, preferably, abutting the hub member **30**, and a second position in which the shield member **40** is extended, such that the puncture tip **28** of the needle cannula **22** is covered by the shield member **40**. In the first position, the needle assembly **16** is in a sampling state with the puncture tip **28** extending from the shield member **40** as shown in FIG. 11, and in the second position, the needle assembly **16** is in a shielded state, with the shield member **40** covering the puncture tip **28**, as shown in FIG. 6.

The shield member **40** includes a housing **42** defining a central bore **44** having a distal opening **46** from which the puncture tip **28** extends when the shield member **40** is in the retracted position. The housing **42** of the shield member **40** extends co-axially about the needle cannula **22** and is in axial alignment with the internal passage **38** defined by the tubular wall **36** of the hub member **30**. The shield member **40** further includes a pair of stabilizers in the form of wings **48**, **50** that extend laterally from the housing **42** at opposing sides thereof. The lateral wings **48**, **50** provide a butterfly-type wing assembly useful for positioning and placement of the needle assembly **16** and blood collection set **10** during a blood collection procedure. The shield member **40** may further include a dorsal wing **52** extending from the housing **42** and located between the lateral wings **48**, **50**. The dorsal wing **52** is preferably symmetrically positioned on the housing **42** between the lateral wings **48**, **50**.

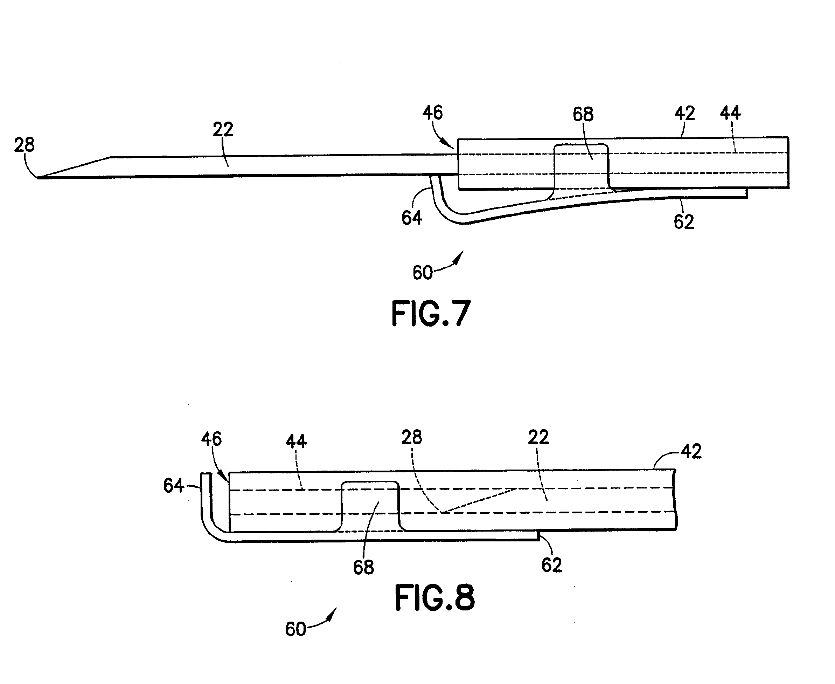

Referring now to FIGS. 1-8, a needle tip guard **60** is optionally attached to the housing **42** of the shield member **40** distally forward of the lateral wings **48**, **50** and dorsal wing **52**. The tip guard **60** is provided to automatically cover the distal opening **46** of the central bore **44** of the housing **42** when the shield member **40** is moved to the extended position covering the puncture tip **28** of the needle cannula **22**. The tip guard **60** is provided as a curved leaf spring of metal or the like having an axially extending spring leg **62**, a generally right-angled locking plate **64**, and a pair of clip legs **66**, **68** forming a gripping collar for securely holding the tip guard **60** onto the housing **42** of the shield member **40**. Operation of the tip guard **60** shown in FIGS. 1-8 will be discussed hereinafter.

As stated previously, the hub member **30** and shield member **40** are generally connected by a pair of extendable members **70**, **100**. The first extendable member **70** is comprised of a pair of folding legs, which include a first or proximal leg **72** and a second or distal leg **74**. The first and second legs **72**, **74** are connected by a hinged knee joint **76** that includes a fingerplate **78.** The first leg **72** of the pair of folding legs is hingedly connected to the hub member **30** and the second leg **74** of the pair of legs is hingedly connected to the shield member **40**. The first leg **72** includes a first end **80** and a second end **82**. The first end **80** of the first leg **72** is connected to the fingerplate **78** by a hinged connection **84**, and the second end **82** of the first leg **72** is connected to the hub member **30** by a hub hinge at hinged connection **86**. Likewise, the second leg **74** includes a first end **90** and a second end **92**. The first end **90** of the second leg **74** is connected by a hinged connection **94** to the fingerplate **78**, and the second end **92** of the second leg **74** is connected to the shield member **40** by a hinged connection **96**. Preferably, the fingerplate **78** of the knee joint **76** is concave to provide a convenient grasping location for the user of the needle assembly **16**.

Similarly, the second extendable member **100** is comprised of a pair of folding legs, which include a first or proximal leg **102** and a second or distal leg **104**. The first and second legs **102**, **104** are connected by a hinged knee joint **106** that includes a fingerplate **108**. The first leg **102** of the pair of folding legs is hingedly connected to the hub member **30** and the second leg **104** of the pair of legs is hingedly connected to the shield member **40**. The first leg **102** includes a first end **110** and a second end **112**. The first end **110** of the first leg **102** is connected to the fingerplate **108** by a hinged connection **114**, and the second end **112** of the first leg **102** is connected to the hub member **30** by a hub hinge at hinged connection **116**. Likewise, the second leg **104** includes a first end **120** and a second end **122**. The first end **120** of the second leg **104** is connected by a hinged connection **124** to the fingerplate **108**, and the second end **122** of the second leg **104** is connected to the shield member **40** by a shield hinge at hinged connection **126**. Preferably, the fingerplate **108** of the knee joint **106** is also concave to provide a convenient grasping location for the user of the needle assembly **16**. The respective folding legs **72**, **74** and **102**, **104** of the first and second extendable members **70**, **100** are configured to generally extend laterally along the lateral sides of the needle assembly **16** when the shield member **40** is moved to the extended position.

The needle assembly **16** further includes a locking assembly **140** located on one of the extendable members **70**, **100** for locking the needle assembly **16** in the shielded state with the shield member **40** in the second, extended position. In particular, the locking assembly **140** is preferably integrally formed as part of, for example, knee joint **106**. The locking assembly **140** is formed by a pair of opposed locking members **142**, **144**. The locking members **142**, **144** extend from the inside surface of fingerplate **108** and extend toward opposing fingerplate **78**. The locking members **142**, **144** each include sloped leading edges **146**, **148**, respectively, located opposite from substantially planar locking edges **150**, **152**, respectively. The locking members **142**, **144** define a recess therebetween configured to receive the needle cannula **22**. The locking members **142**, **144** are generally configured to engage the needle cannula **22** when the shield member **40** is moved to the extended position. Accordingly, the locking members **142**, **144** are preferably made resiliently flexible so that the needle cannula **22** is able to separate the locking members **142**, **144** as the shield member **40** is moved to the extended position. The locking assembly **140** may also be located on the fingerplate **78** of knee joint **76**. The operation of the locking assembly **140** will be discussed more fully hereinafter.

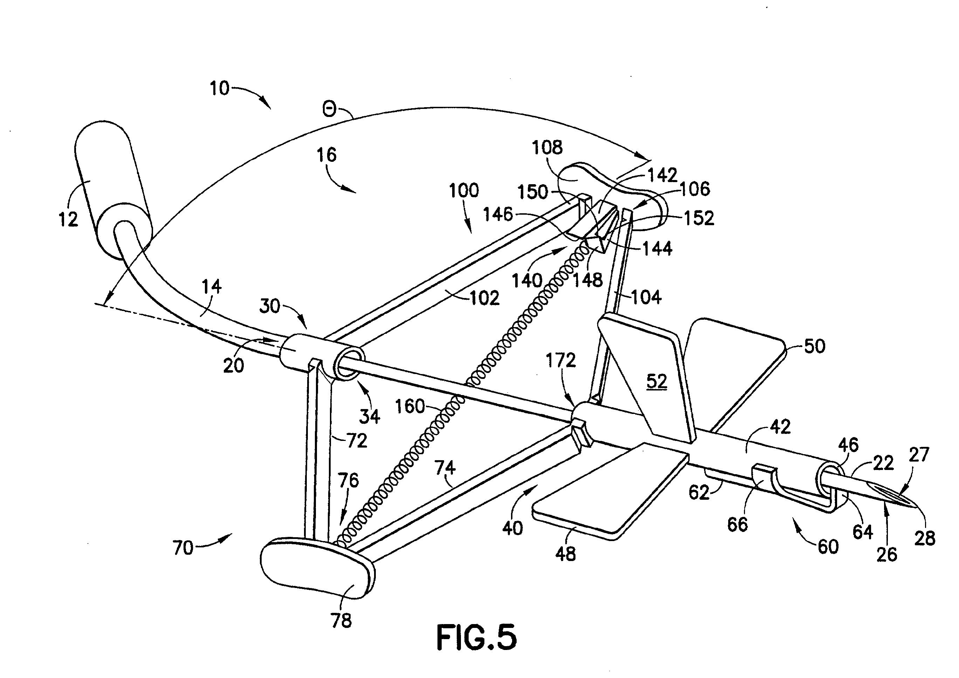

The needle assembly **16** further includes a tension spring **160** operatively connected between the fingerplates **78**, **108** of the respective hinged knee joints **76**, **106**. In particular, the tension spring **160** is connected between inside surfaces of the fingerplates **78**, **108** by means customary to the art. For example, the tension spring **160** may be mechanically attached by fasteners inside the surface of the fingerplates **78**, **108** at the proximal ends of the fingerplates **78**, **108**. Other equivalent means may be used to attach the tension spring **160** to the fingerplates **78**, **108**, such as by an adhesive, and are within the scope of the present invention. The tension spring **160** is preferably adapted to provide forces that continuously bias the fingerplates **78**, **108** toward one another. Accordingly, with the hub member **30** located in the position shown in FIG. 1, the tension spring **160** biases the hub member **30** into engagement with the shield member **40** through the proximal legs **72**, **102** of the respective extendable members **70**, **100**. Once the hub member **30** is moved to a position overlapping the tension spring **160**, the energy of the tension spring **160** is used to move the shield member **40** to the extended position and further draws the fingerplates **78**, **108** toward one another as shown, for example, in FIG. 5 as discussed hereinafter.

A removable, protective needle cover **170** may be used to cover the distal end **26** of the needle cannula **22** and, more particularly, the puncture tip **28** of the needle cannula **22**. The needle cover **170** is preferably positioned over the puncture tip **28** of the needle cannula **22** in a pre-use state of the needle assembly **16**, wherein the shield member **40** is in the retracted position and maintained in the retracted position by the tension spring **160**. The needle cover **170** is preferably an inexpensive, elongated plastic needle cover such as those commonly used as a needle protector in the medical field.

With the basic structure of the blood collection set **10** and needle assembly **16** described, operation of the blood collection set **10** and needle assembly **16** will be described with continued reference to FIGS. 1-8. The needle assembly **16** is preferably provided in the sampling state with the shield member **40** in the first, retracted position and the needle cover **170** positioned over the distal end **26** of the needle cannula **22**. The shield member **40** is held in the first retracted position by the tension spring **160**. Referring to FIG. 1, the tension spring **160** biases the fingerplates **78**, **108** toward one another. This force is transmitted through the proximal legs **72**, **102** of the respective extendable members **70**, **100** to the hub member **30**. The force acting on the hub member **30** causes the hub member **30** to engage or abut the proximal end **172** of the shield member **40**, with the hub hinges at hinged connections **86** and **116** positioned in front of or distal to the tension spring **160**. With the shield member **40** held in the first position by the tension spring **160**, a blood collection tube may be fixed to the fixture **12** located at the first end **18** of the flexible tube **14**.

To use the blood collection set **10** and needle assembly **16** in a medical procedure, the user of the blood collection set **10** will first sterilize the intended area of puncture on the patient's body and remove the needle cover **170** from the distal end **26** of the needle cannula **22**. The user of the needle assembly **16** may then grasp the lateral wings **48**, **50** and the dorsal wing **52** to assist in positioning the needle assembly **16** at the intended area of puncture on the patient's body. The lateral wings **48**, **50** and the dorsal wing **52** are preferably made flexible so that they may be folded together to provide a convenient handle for manipulating the needle assembly **16**. Once the puncture tip **28** of the needle cannula **22** is inserted into a blood vessel in the patient's body (i.e., venipuncture), the user may spread the lateral wings **48**, **50** flat onto the patient's body and tape them in place to maintain the positioning and placement of the needle assembly **16** during the blood collection procedure or other medical procedure. The lateral wings **48**, **50** in this configuration will also provide a barrier between the needle cannula **22** and the user's fingertips, which will help prevent an accidental needle-stick should the needle cannula **22** inadvertently retract from the site of insertion. The user of the needle assembly **16** may also grasp the fingerplates **78**, **108** attached to the extendable members **70**, **100** with his or her free hand to further assist in positioning and placing the needle assembly **16** at the intended site of insertion into the patient's body.

After completing venipuncture, the user of the needle assembly **16** will typically collect one or more blood samples by attaching one or more blood collection tubes (not shown) to the fixture **12**. Once the blood collection step is completed, the user of the blood collection set **10** and needle assembly **16** may then actuate the needle assembly **16** from the sampling state to the shielded state. To actuate the needle assembly **16**, the user of the needle assembly **16** grasps the hub member **30** and begins to move the hub member **30** proximally away from the shield member **40**. As the user initially moves the hub member **30** axially away from the shield member **40**, the tension spring **160** continues to act upon the hub member **30** to bias the hub member **30** toward the shield member **40**. The user must overcome this force to begin moving the hub member **30** away from the shield member **40**.

Once the hub member **30** reaches a point when the proximal legs **72, 102** connected to the hub member **30** for each of the extendable members **70**, **100** forms an angle Θ of greater than 90° with the central axis of the needle cannula **22,** the force of the tension spring **160** is released to automatically bias the hub member **30** and the shield member **40** away from each other in opposing axial directions to the second position. In particular the tension spring **160** is generally provided as an "over-center" spring arrangement such that when the hub member **30** is moved to a position generally overlapping the tension spring **160**, the force of the tension spring **160** in released to move the fingerplates **78**, **108** toward one another. The overlapping position with respect to the tension spring **160** occurs approximately when the proximal legs **72**, **102** of the respective extendable members **70, 100** form the angle Θ greater than 90° with the central axis of the needle cannula **22**. Once the tension spring **160** is moved over-center, the tension spring **160** automatically biases the fingerplates **78**, **108** toward one another, which simultaneously biases the shield member **40** and the hub member **30** axially apart. The released force of the tension spring **160** is transmitted through the proximal legs **72**, **102** and the distal legs **74, 104** of the respective extendable members **70, 100** to move the shield member **40** and the hub member **30** axially apart. As the shield member **40** and the hub member **30** moves axially away from each other, the needle cannula **22** is withdrawn into the shield member **40**. Thus, the tension spring **160** is used to automatically (i.e., passively), move the shield member **40** to the extended position.

The tension spring **160** may have a spring constant and free length that is sufficient to move to the shield member **40** to an at least partially extended position while the needle cannula **22** is in the patient's body without entirely removing the needle cannula **22** from the patient's body. For example, the tension spring **160** may be limited in strength such that as the user of the needle assembly **16** moves the hub member **30** to the position where the proximal legs **72, 102** form the angle Θ of greater than 90° with the needle cannula **22**, the tension spring **160** would have sufficient force to begin moving the shield member **40** to the extended position, but have insufficient force to entirely remove the needle cannula **22** from the patient's body. The user may then completely withdraw the needle cannula **22** from the patient's body. Thereafter, the tension spring **160** provides sufficient force acting on the hub and shield members **30, 40** to move the shield member **40** to the extend position covering the puncture tip **28** of the needle cannula **22**.

Alternatively, the user may elect to entirely withdraw the needle cannula **22** from the patient's body before actuating the needle assembly **16**. After completely withdrawing the needle cannula **22** from the patient's body, the user of the needle assembly **16** maintains the position of the shield member **40** with one hand and moves the hub member **30** axially away from the shield member **40** to the position where the proximal legs **72, 102** of the extendable members **70, 100** form an angle Θ of greater than 90° with the needle cannula **22**, which releases the tension spring **160** to bias the fingerplates **78, 108** toward one another and move the shield member **40** to the extended position.

In either method of operation discussed hereinabove, as the shield member **40** moves forward toward the second extended position, the locking members **142, 144** extending from fingerplate **108** of extendable member **100** move toward the needle cannula **22**. As the shield member **40** travels along the needle cannula **22** toward the extended position, the sloped leading edges **146, 148** of the locking members **142, 144** slidably engage onto the needle cannula **22**. As the shield member **40** reaches the fully extended position, the locking edges **150, 152** of the locking members **142, 144** snap into engagement onto the needle cannula **22** to secure the shield member **40** in the extended position. Once the locking members **142, 144** are engaged onto the needle cannula **22**, the extendable members **70, 100** will be prevented from moving transversely away from the lateral sides of the needle assembly **16**. The tension spring **160** further prevents the extendable members **70, 100** from moving away from each other. Thus, the locking assembly **140**, once engaged, prevents the re-emergence of the puncture tip **28** of the needle cannula **22** from the shield member **40**.

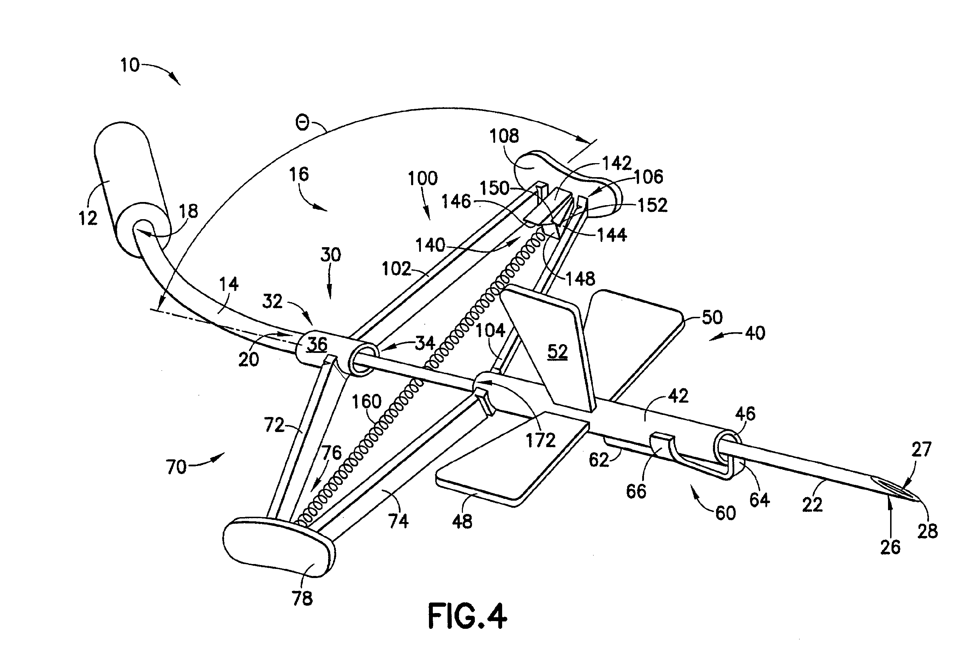

The tip guard **60** attached to the housing **42** of the shield member **40** will automatically cover the distal opening **46** of the central bore **44** once the needle cannula **22** is fully covered by the shield member **40**. In particular, when the shield member **40** is in the retracted position (as in FIG. 1), the needle cannula **22** extends outward from the housing **42** of the shield member **40** and the locking plate **64** of the tip guard **60** is biased into engagement with the bottom surface of the needle cannula **22**. The spring leg **62** biases the locking plate **64** into engagement with the needle cannula **22**. As the needle cannula **22** begins to be covered by the shield member **40**, as shown in FIGS. 4 and 5, the locking plate **64** slides along the bottom surface of the needle cannula **22** until the needle cannula **22** is fully covered by the shield member **40**. Once the locking plate **64** is no longer in engagement with the needle cannula **22**, the axially extending spring leg **62** causes the locking plate **64** to automatically extend over the distal opening **46** of the central bore **44** of the housing **42**, as shown in FIG. 6. The tip guard **60** fully covers the distal opening **46** of the central bore **44** of the housing **42**, thereby preventing any re-emergence of the needle cannula **22**. With the blood collection set **10** and needle assembly **16** now placed in a safety state, a blood collection tube (not shown) may be safely removed from the needle assembly **16**, and the needle assembly **16** disposed of as medical waste.

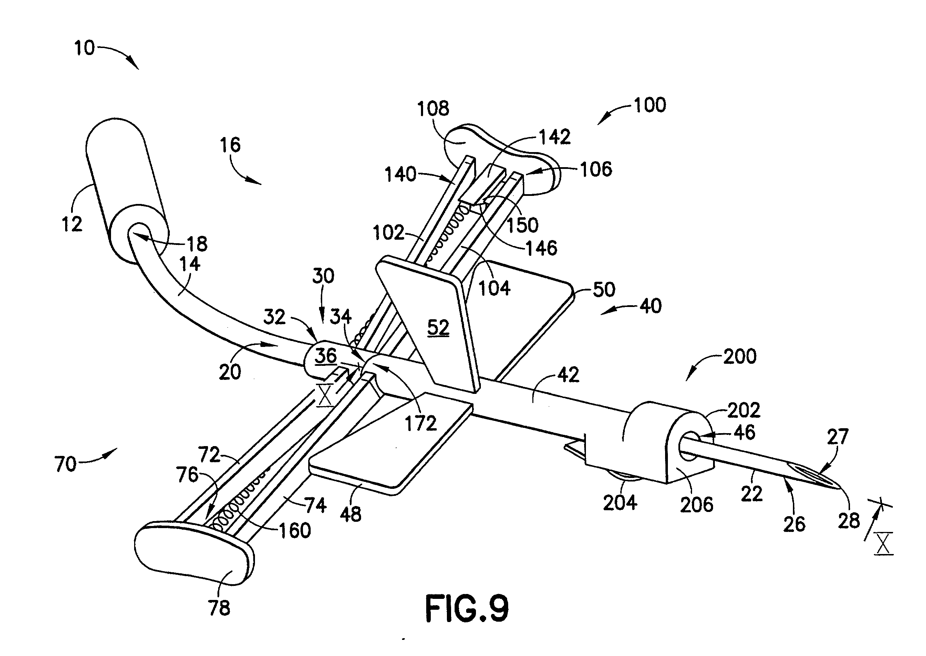

Referring to FIGS. 9-11, a further modification to the blood collection set **10** and needle assembly **16** of FIGS. 1-6 is shown. The blood collection set **10** and needle assembly **16** of FIGS. 9-11 are substantially similar to the blood collection set **10** and needle assembly **16** of FIGS. 1-6, but further include a modified needle tip guard **200.** The tip guard **200** is generally comprised of a tip guard housing **202** and a protective clip **204.** The tip guard housing **202** is preferably a unitary structure molded from a thermoplastic material. The tip guard housing **202** may be formed integrally with the housing **42** of the shield member **40** as shown in FIGS. 9-11, or formed separately from and attached to the housing **42** of the shield member **40**. The tip guard housing **202** includes a distal end **206** and an internal passage **208** extending through the tip guard housing **202** to cooperate with the central bore **44** of the shield member **40**. Portions of the internal passage **208** adjacent the distal end **206** define an enlarged clip receptacle or recess **210**. A clip mounting post **212** extends downward from the tip guard housing **202**. The protective clip **204** is preferably unitarily stamped and formed from a resiliently deflectable metallic material. The protective clip **204** includes a spring leg **214** with a proximal end **216** and an opposed distal end **218**. A mounting aperture (not shown) extends through the spring leg **214** at a location near the proximal end **216**. The mounting aperture has a diameter approximately equal to or slightly less than the diameter of the mounting post **212** of the tip guard housing **202**. As such, the mounting post **212** can be forced through the mounting aperture when the axis of the mounting post **212** and the axis of the mounting aperture are substantially co-linear. A lockout leg **220** extends angularly from the distal end **218** of the spring leg **214**. The lockout leg **220** is bent back toward the proximal end **216** of the spring leg **214**.

The modified tip guard **200** operates as follows. When the shield member **40** is in the retracted position, the lockout leg **220** is biased against the needle cannula **22** by the spring leg **214**, as shown in FIG. 10. When the hub member **30** is moved to the position where the proximal legs **72**, **102** of the extendable members **70**, **100** form the angle Θ of greater than 90° with the needle cannula **22,** the tension spring **160** automatically (i.e., passively) moves the shield member **40** axially away from the hub member **30** to the extended position. Simultaneously, the needle cannula **22** is withdrawn into the housing **42** of the shield member **40**. During the movement of the shield member **40** axially away from the hub member **30**, the lockout leg **220** remains biased in contact with the needle cannula **22** by the spring leg **214**. As the shield member **40** reaches its fully extended position, the needle cannula **22** withdraws axially past the recess **210** formed by the tip guard housing **202**. Because the lockout leg **220** is spring-biased toward the recess **210** by the spring leg **214**, as soon as the needle cannula **22** is withdrawn past the lockout leg **220,** the lockout leg **220** is spring-biased into the recess **210**. The lockout leg **220** thereafter prevents re-emergence of the needle cannula **22** from the tip guard housing **202** of the needle point guard **200**.

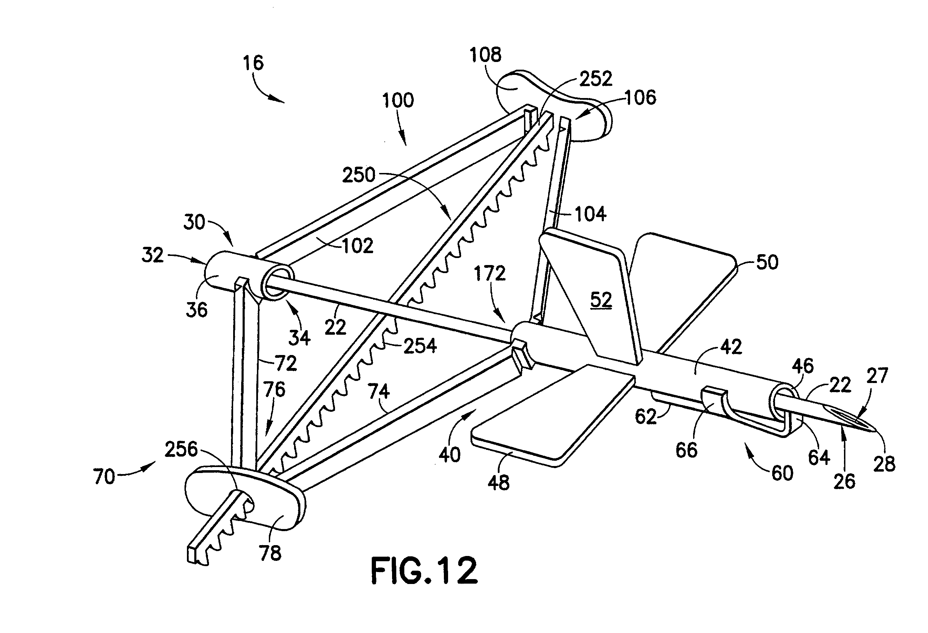

A modification to the blood collection set **10** and needle assembly **16** of FIGS. 1-6 is shown in FIG. 12. The blood collection set **10** and needle assembly **16** of Fig. 12 are substantially similar to the blood collection set **10** and needle assembly **16** of FIGS. 1-6, but no longer require the tension spring **160**. Because the tension spring **160** is omitted in the blood collection set **10** and needle assembly **16** of FIG. 12, the needle assembly **16** must be manually actuated to move the shield member **40** to the extended position. This is accompanied by pressing on the fingerplates **78, 108** of the respective hinged knee joints **76, 106**, which will transfer this applied force to the extendable members **70, 100**. The extendable members **70, 100** through the proximal legs **72, 102** and distal legs **74, 104** will urge the hub and shield members **30, 40** apart. The shield member **40** will ultimately reach the extended position as shown in FIG. 6, discussed previously.

The blood collection set **10** and needle assembly **16** of FIG. 12 differs from the blood collection set **10** and needle assembly **16** discussed previously in that a modified locking assembly **250** is provided. The modified locking assembly **250** is comprised of a locking member **252** having a plurality of ratchet teeth **254** depending therefrom. The locking member **252** extends between the knee joints **76**, **106** of the extendable members **70, 100**. In particular, the locking member **252** extends from one of the fingerplates **78, 108** to the opposing fingerplates **78, 108.** In FIG. 12, the locking member **252** extends from fingerplate **108** of extendable member **100** to the opposing fingerplate **78** of extendable member **70**, but this configuration may be reversed. The locking member **252** extends through an opening or aperture **256** formed in fingerplate **78**. The ratchet teeth **254** are preferably configured such that as inward acting force is applied to the respective fingerplates **78, 108**, the ratchet teeth **254** permit the opposing fingerplates **78, 108** to move uni-directionally toward one another. As the fingerplates **78, 108** are moved toward one another, the extendable members **70, 100**, through their respective proximal legs **72, 102** and distal legs **74, 104**, move the shield member **40** and the hub member **30** apart. The ratchet teeth **254** are preferably configured to prevent the locking member **252** from withdrawing from the aperture **256** (i.e., move bi-directionally) in fingerplate **78**, which prevents the shield member **40** from retracting axially toward the hub member **30.** Thus, the locking member **252** and the ratchet teeth **254** permit one-directional movement only. The embodiment of the present invention shown in FIG. 12 may include the needle tip guard **60** or the modified needle tip guard **200**, both of which were discussed previously. All other aspects of the blood collection set **10** and needle assembly **16** in FIG. 12 are substantially similar to the blood collection set **10** and needle assembly **16** shown in FIGS. 1-6.

While the blood collection set and shieldable needle assembly of the present invention have been described with respect to preferred embodiments, various modifications and alterations of the present invention may be made without departing from the spirit and scope of the present invention. The scope of the present invention is defined in the appended claims and equivalents thereto.

## Claims

1. A shieldable needle assembly, comprising:
a needle cannula having a proximal end and a distal end with a puncture tip;
a hub member supporting the proximal end of the needle cannula;
a shield member including a housing defining a central bore, the shield member in axial alignment with the hub member and extending co-axially about the needle cannula;
a pair of extendable members connecting the hub member and the shield member, with the extendable members each including a pair of folding legs connected by a hinged knee joint, and with one leg of the pair of legs for each extendable member hingedly connected to the hub member at a hub hinge and the other leg of the pair of legs for each extendable member hingedly connected to the shield member; and
a tension spring operatively connected between the extendable members,
wherein the hub member and the shield member are adapted for relative axial movement with respect to each other in opposing axial directions between a first position in which the puncture tip of the needle cannula is exposed and a second position in which the shield member covers the puncture tip of the needle cannula, wherein the hub hinge is located in front of the tension spring when the hub member and the shield member are in the first position, and wherein when the hub member is moved in an axial direction away from a proximal end of the shield, the legs connected to the hub member for each extendable member are pivoted to a position forming an angle of greater than 90° with the axis of the needle cannula, thereby causing the shield member and the hub member to be biased in opposing axial directions to the second position by the tension spring.

2. A needle assembly as in claim 1, wherein the shield member includes a pair of butterfly wings extending laterally from opposing sides of the housing of the shield member.

3. A needle assembly as in claim 2, wherein the housing of the shield member further includes a dorsal wing located between the butterfly wings.

4. A needle assembly as in claim 1, wherein the shield member includes a tip guard for protectively surrounding the puncture tip of the needle cannula when the shield member is moved to the second position, and wherein the tip guard comprises a tip guard housing and a spring clip connected to the tip guard housing, with the spring clip biased against the needle cannula when the shield member is in the first position and resiliently extends over the puncture tip of the needle cannula when the shield member is movable to the second position.

5. A needle assembly as in claim 1, further including a tip guard attached to the housing of the shield member and comprising a spring leg extending axially along the housing, a pair of clip legs securing the tip guard to the housing, and a locking plate resiliently biased against the needle cannula when the shield member is in the first position and resiliently extends over a distal opening to the central bore of the housing when the shield member is moved to the second position.

6. A needle assembly as in claim 1, wherein the knee joint for each extendable member further includes a fingerplate to enable the user to manipulate the assembly.

7. A needle assembly as in claim 1, further comprising a locking assembly extending from the fingerplate of at least one of the extendable members toward the needle cannula and configured to engage the needle cannula such that when the shield member and the hub member are moved in opposing axial directions to the second posotion by the tension spring, the locking member automatically engages onto the needle cannula to secure the shield member in the second position.

8. A needle assembly as in claim 7, wherein the locking assembly is comprised of a pair of opposed locking members defining a recess therebetween for receiving the needle cannula.

9. A needle assembly as in claim 8, wherein the locking members are made of a resiliently flexible material such that the needle cannula is able to separate the locking members under the force provided by the tension spring.

10. A needle assembly as in claim 1, wherein the hub member is adapted for connection to a flexible tube of a blood collection set.

11. A needle assembly as in claim 1, further including a removable protective cover positioned over the distal end of the needle cannula.

12. A shieldable blood collection set, comprising:
a flexible tube having opposed first and second ends, with the first end of the flexible tube adapted for connection to a receptacle;
a hub member connected to the second end of the flexible tube;
a needle cannula having a proximal end and a distal end with a puncture tip, the proximal end connected to the hub member and the distal end projecting from the hub member, the needle cannula defining a lumen in fluid communication with the flexible tube and the fixture;
a shield member including a housing defining a central bore, the shield member in axial alignment with the hub member and extending co-axially about the needle cannula;
a pair of extendable members connecting the hub member and the shield member, the extendable members each including a pair of folding legs connected by a hinged knee joint, and with one leg of the pair of legs for each extendable member connected hingedly to the hub member at the hub hinge and the other leg of the pair of legs for each extendable member connected hingedly to the shield member; and
a tension spring operatively connected between the extendable members,
wherein the hub member and the shield member are adapted for relative axial movement with respect to each other in opposing axial directions between a first position in which the puncture tip of the needle cannula is exposed and a second position in which the shield member covers the puncture tip of the needle cannula, wherein the hub hinge is located in front of the tension spring when the hub member and the shield member are in the first position, and wherein when the hub member is moved in an axial direction away from a proximal end of the shield member, the legs connected to the hub member for each extendable member are pivoted to a position forming an angle of greater than 90° with the axis of the needle cannula, thereby causing the shield member and the hub member to be biased in opposing axial directions to the second position by the tension spring.

13. A shieldable blood collection set as in claim 12, wherein the shield member includes a pair of butterfly wings extending laterally from opposing sides of the housing of the shield member.

14. A shieldable blood collection set as in claim 1, wherein the shield member further includes a dorsal wing located between the butterfly wings.

15. A shieldable blood collection set as in claim 12, wherein the shield member includes a tip guard for protectively surrounding the puncture tip of the needle cannula when the shield member is in the second position, and wherein the tip guard comprises a tip guard housing and a spring clip connected to the tip guard housing, with the spring clip biased against the needle cannula when the shield member is in the first position and resiliently extends over the puncture tip of the needle cannula when the shield member is moved to the second position.

16. A needle assembly as in claim 1, further including a tip guard attached to the housing of the shield member and comprising a spring leg extending axially along the housing, a pair of clip legs securing the tip guard to the housing, and a locking plate resiliently biased against the needle cannula when the shield member is in the retracted position and resiliently extends over a distal opening to the central bore of the housing when the shield member is moved to the second position.

17. A shieldable blood collection set as in claim 12, wherein the knee joint for each extendable member further includes a fingerplate to enable the user to manipulate the needle assembly.

18. A shieldable blood collection set as in claim 12, further comprising a locking assembly extending from the fingerplate of at least one of the extendable members toward the needle cannula and configured to engage the needle cannula such that when the shield member and the hub member are moved in opposing axial directions to the second position by the tension spring, the locking assembly automatically engages onto the needle cannula to secure the shield member in the second position.

19. A shieldable blood collection set as in claim 18, wherein the locking assembly is comprised of a pair of opposed locking members defining a recess therebetween for receiving the needle cannula.

20. A shieldable blood collection set as in claim 19, wherein the locking members are made of a resiliently flexible material such that the shaft of the needle cannula is able to separate the locking members under the force provided by the tension spring.

21. A shieldable blood collection set as in claim 12, further including a removable protective cover positioned over the distal end of the needle cannula.

22. A method of automatically placing a shieldable needle assembly into a safety state in which a puncture tip of a needle cannula is covered by the assembly, comprising the steps of:
providing a shieldable needle assembly, comprising:
a needle cannula having a proximal end and a distal end with a puncture tip;
a hub member supporting the proximal end of the needle cannula;
a shield member including a housing defining a central bore, with the shield member in axial alignment with the hub member and extending co-axially about the needle cannula, and with the shield member and the hub member axially movable with respect to each other between a first position in which the puncture tip of the needle cannula is exposed and an a second position in which the shield member covers the puncture tip of the needle cannula;
a pair of extendable members connecting the hub member and the shield member, with the extendable members each including a pair of folding legs connected by a hinged knee joint, and with one leg of the pair of legs for each extendable member hingedly connected to the hub member at a hub hinge and the other leg of the pair of legs for each extendable member hingedly connected to the shield member; and
a tension spring operatively connected between the extendable members for moving the shield member and the hub member in opposing axial directions, to the extended position,
wherein the hub hinge is located in front of the tension spring when the hub member and the shield member are in the first position;
using the shieldable needle assembly in a medical procedure; and
moving the hub member in an axial direction away from a proximal end of the shield member until the legs connected to the hub member for each extendable member are pivoted to a position forming an angle of greater than 90° with the axis of the needle cannula such that the shield member and the hub member are biased in opposing axial directions to the second position by the tension spring.

23. A method as in claim 22, wherein the knee joint for each extendable member further includes a fingerplate, and the needle assembly further comprises a locking assembly extending from the fingerplate of at least one of the extendable members toward the needle cannula, with the locking assembly having a pair of opposed locking members configured to engage the needle cannula and defining a recess therebetween for receiving the needle cannula,
wherein the method further comprises the step of engaging the locking members onto the needle cannula when the shield member is biased to the second position by the tension spring.

24. A shieldable needle assembly, comprising:
a needle cannula having a proximal end and a distal end with a puncture tip;
a hub member fixedly supporting the proximal end of the needle cannula;
a shield member including a housing defining a central bore, with the shield member in axial alignment with the hub member and extending co-axially about the needle cannula, and with the shield member movable between a retracted position in which the puncture tip of the needle cannula is exposed and an extended position in which the shield member covers the puncture tip of the needle cannula;
a pair of opposing extendable members connecting the hub member and the shield member, with the extendable members each including a pair of folding legs connected by a hinged knee joint, and with one leg of the pair of legs for each extendable member hingedly connected to the hub member at a hub hinge and the other leg of the pair of legs for each extendable member connected hingedly to the shield member; and
a locking assembly connecting the knee joints of the extendable members and comprised of a locking member having a plurality of ratchet teeth, with the ratchet teeth of the locking member configured to permit uni-directional movement of the knee joints of the extendable members toward one another.

25. The shieldable needle assembly of claim 24, wherein the knee joint for each extendable member further includes a fingerplate, and wherein the locking member extends from one fingerplate to the fingerplate of the knee joint of the opposing extendable member.
